# EUROPEAN PATENT SPECIFICATION

(11) **EP 1 367 951 B1**
(45) Date of publication and mention of the grant of the patent: **14.11.2007**
(21) Application number: 02712585.5
(22) Date of filing: 20.02.2002
(51) Int. Cl.: A61B 17/86, A61F 2/30, A61F 2/36

(54) **BONE SCREW AND METHOD FOR PRODUCING THE THREADS THEREOF**
KNOCHENSCHRAUBE UND VERFAHREN ZUR HERSTELLUNG DES GEWINDES DAFÜR
VIS A OS ET PROCEDE POUR REALISER SES FILETS

(30) Priority: 21.02.2001 SE 0100573
(43) Date of publication of application: 10.12.2003
(73) Proprietor: Hansson, Henrik, 590 77 Vreta Kloster (SE)
(72) Inventor: Hansson, Henrik, 590 77 Vreta Kloster (SE)
(74) Representative: Wagner, Karl Heinz
(86) International application number: PCT/SE2002/000288
(87) International publication number: WO 2002/065925

(56) References cited:
- EP-A2- 0 441 577
- EP-A2- 0 820 731
- DE-U1- 29 522 089
- US-A- 5 403 136
- US-A- 5 605 457
- US-A- 5 743 914
- US-A- 5 779 704
- US-A- 5 836 950
- US-B1- 6 224 606

## Description

The present invention relates to a bone screw for implants for fixation of bone fragments at fractures wherein the bone screw comprises a threaded or tapped part which is adapted to be screwed into a bone fragment at the fracture. The threaded part extends backwards from a front end portion of the bone screw to an untapped part thereof and has threads with the same outer diameter. The threads in the threaded part extend from a core and the threads of the threaded part have the same pitch of thread. The invention also relates to a method for producing the threads of the bone screw.

Bone screws for bone implants (so called lag screws) for fixation of bone fragments at femoral fractures are previously known from e.g. US 5 836 950. At such bone screws, the width of the grooves between the threads is the same along the entire part. This design is not optimal for stable anchoring to surrounding bone material, particularly not if said bone material is osteoporotic.

There are also prior art bone screws in which the threaded part has a cylindrical core. For being able to screw such a bone screw into bone material, one normally has to pre-drill said bone material for obtaining a hole therein for the cylindrical core or one has to use a special bone screw which at the front is provided with a drill bit for obtaining said hole.

At prior art bone screws, the threads are normally designed such that one has to cut threads in the bone material for the threads of the bone screw either by means of a separate thread tap or a thread tap which is built into the bone screw.

The object of the present invention has been to provide a bone screw with improved properties for engaging bone material particularly if this is osteoporotic and also in order to obviate predrilling of the bone material for the core if said bone material is osteoporotic. This is arrived at according to the invention by providing the bone screw with the characterizing features of subsequent claim 1.

The object of the invention is also a method for providing the threads of the bone screw and this method includes the characterizing measures of subsequent claim 17.

Since the core of the bone screw according to the characterizing features of claim 1 is conical, it can be screwed into spongy bone material without pre-drilling thereof for the core. Also, the conical core functions as a "plough" which presses the surrounding bone material in radially outwards direction, i.e. it compresses the bone material closest to the threaded part of the bone screw.

Since the bone screw according to said characterizing features of subsequent claim 1 further comprises a threaded part with threads having truncated crests, the width of which increases in backwards direction, i.e. with a thin thread at the front and thicker threads further backwards, it is possible to cut threads in the surrounding bone material directly with the threads of the bone screw instead of doing it with a separate thread tap or a thread tap built into the bone screw.

Since the width of the groves between the threads decreases because of the increase in width of the thread crests, it is achieved that the surrounding bone material is compressed by the threads when the bone screw is screwed into said bone material.

A further advantage with a conical core, that the threads are thin at the front and increase gradually in width and that the width of the grooves between the threads decreases, is that the bone screw gets a firm grip also in osteoporotic bone material when it is screwed into such bone material.

The invention will be further described below with reference to the accompanying drawings, in which
figure 1 illustrates a bone screw according to the invention in cooperation with a bone implant which is shown partly in section and adapted for fixation of bone fragments at a femoral fracture;
figure 2 illustrates front parts of a bone screw according to the invention in a large scale;

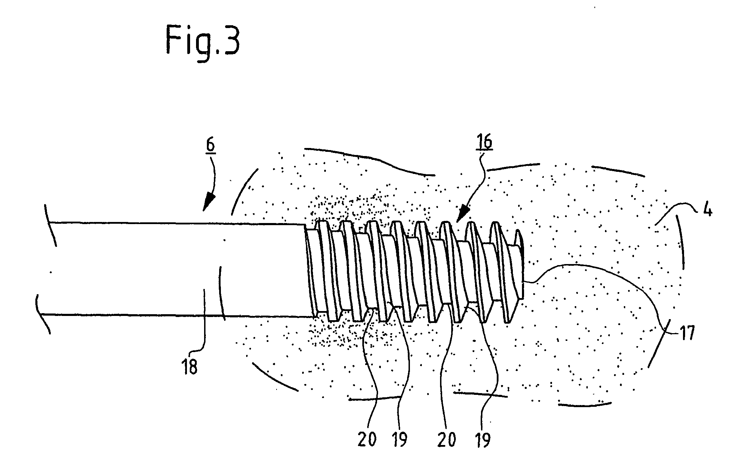

figure 3 illustrates front parts of a bone screw according to figure 2 screwed into a bone fragment;
figure 4 illustrates front parts of a bone screw of the same type as in figures 1-3, but with left-hand threads instead of right-hand threads;
figure 5 illustrates front parts of a bone screw according to figures 1-3 and schematically a milling tool for milling the threads of the bone screw; and
figure 6 illustrates a drill in a bone fragment after drilling of a hole for a bone screw therein.

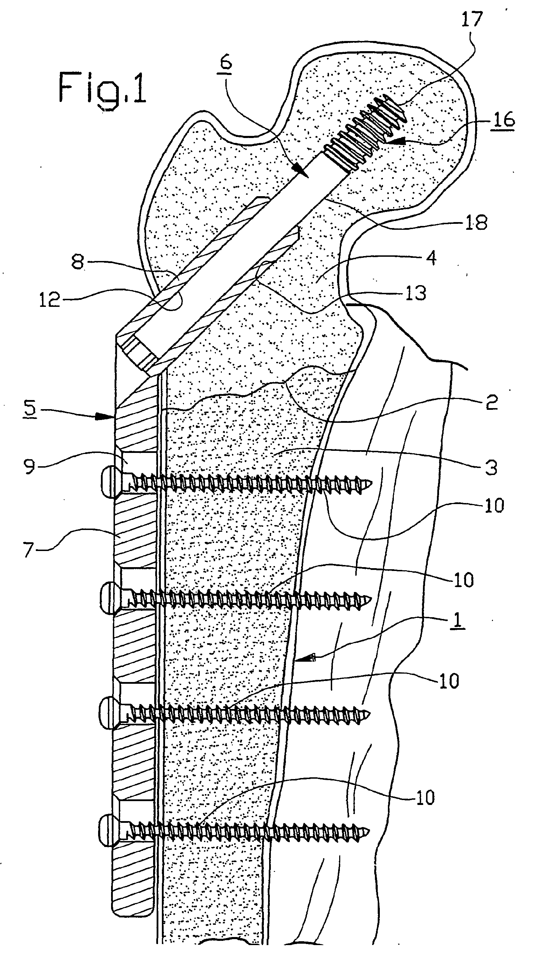

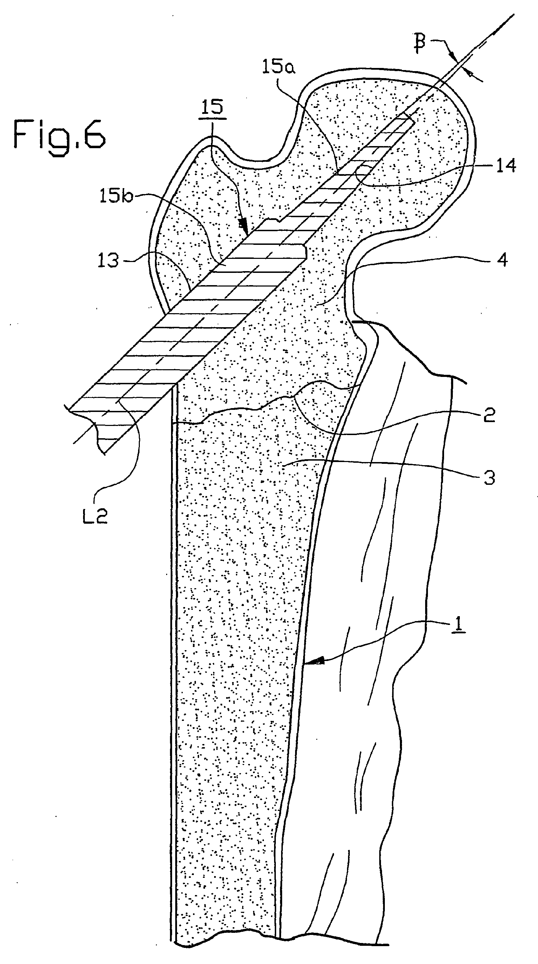

Figures 1 and 6 schematically illustrate a thighbone (femur), the femoral shaft 1 of which has a femoral fracture 2, more closely, in the illustrated example, a sub-trochanteric fracture. The bone fragment 3 beneath the femoral fracture 2 includes substantial parts of the femoral shaft 1, while the bone fragment 4 above the femoral fracture 2 includes the femoral head. For fixation of the bone fragments 3, 4 relative to each other, various types of implants can be used, e.g. an implant 5 as illustrated in figure 1 and in connection therewith a bone screw 6 (so called lag screw) is used.

The implant 5 comprises a plate 7 or a corresponding member and a sleeve 8 or a corresponding member. The plate 7 has four or another suitable number of long holes 9 for screws 10 through which the plate 7 is attached or secured to the lower bone fragment 3. The long holes 9 are located and the screws 10 positioned therein such that the screws 10 and the lower bone fragment 3 can move in upwards direction towards the upper bone fragment 4. The sleeve 8 is inserted into a hole 13 in the upper bone fragment 4 and it has a hole 12 for the bone screw 6, which is secured to the upper bone fragment 4. The sleeve 8 can slide in its longitudinal direction relative to the bone screw 6 and said bone screw 6 as well as the sleeve 8 are in a manner known per se designed such that the bone screw 6 can not rotate relative to the sleeve 8 when it is inserted into said sleeve 8. Since embodiments for preventing said rotation are known, they have not been illustrated in the drawings. Eventually, there may be a stop screw (not shown) for preventing or limiting, when necessary, sliding of the sleeve 8 relative to the bone screw 6.

The hole 13 is pre-drilled by means of a drill 15. Eventually, the drill 15 may be a so called stepped drill with a rear portion 15b for drilling the hole 13 for the sleeve 8 and a front portion 15a for drilling a hole 14 for a core 21 of a threaded or tapped part 16 of the bone screw 6. It is however, not always necessary to drill the hole 14 since the threaded part 16 is designed to be screwed into the bone material if said bone material is osteoporotic.

Specifically, the method for application of the bone screw 6 normally starts with drilling, in a manner known per se, a thin hole (e.g. having a diameter of 3,2 mm) for a guide wire (not shown). Then, the thin hole is drilled into the hole 13 having a larger diameter for an untapped part 18 of the bone screw 6 and if the spongy parts of the bone material are not osteoporotic, the thin hole is drilled also into the hole 14 for the threaded part 16 but with a less diameter than the hole 13. If the bone material is osteoporotic, the inner portions of the thin hole is not drilled such that the hole 14 is formed. Said guide wire is removed when it is no longer needed.

The bone screw 6 has a front end portion 17 and its threaded part 16 extends backwards therefrom to the untapped part 18. The threaded part 16 comprises threads 19 and grooves 20 between the threads. The threads 19 have the same or substantially the same outer diameter YD along the threaded part 16.

The threads 19 extend from a core 21 having a circular cross section and extending along parts of or preferably the entire length L of the threaded part 16. At least front parts of the core 21 are conical and the least diameter MD of the core 21 is situated frontmost at the front end portion 17. The diameter of the core 21 increases gradually in backwards direction and it has its largest diameter SD at the transition 22 between the threaded part 16 and the untapped part 18. Each longitudinal side of the core 21 defines e.g. an angle of inclination α of 2-4° with the longitudinal axis L1 of the threaded part 16. At the bone screw 6 shown in the drawings, the angle α is 3°.

The shape of the core 21 can be conical along the entire length L of the threaded part 16, as is shown in the drawings, but the shape of the core 21 may alternatively be conical along only a part of this length L. An example of a particular embodiment of the core 21 is that it has a conical front part, a cylindrical part behind the conical front part and behind the cylindrical part another conical part which extends backwards to the untapped part 18 or terminates in its vicinity.

The threads 19 of the threaded part 16 have the same thread pitch P; the bone screw 6 illustrated in the drawings has e.g. a thread pitch of 3,2 at an outer diameter YD of 12,7 mm. The thread pitch of the bone screw 6 may e.g. be found within an interval of 3,0 at an outer diameter YD of 8,0 mm and 2,6 at an outer diameter of 6,5 mm. The bone screw with said latter pitch may be adapted for external fixation.

At least some of the threads 19 have truncated crests 23 and these crests have truncations 24 that are planar or have another shape. The width b1-b7 of at least some of these crests 23 increases in backwards direction from the front end portion 17. Preferably, the width b1-b7 of the crests increases gradually such that the crest 23 of the first thread 19 closest to the front end portion 17 is narrow, while the crests 23 of the following threads 19 in backwards direction become wider and wider.

Since the threads 19 have the same pitch P, the width B1-B6 of at least some of the grooves 20 between said threads will, in backwards direction, decrease because the width b1-b7 of the crests 23 increases.

This decrease or reduction of the width B1-B6 of the grooves 20 between the threads 19 means that a first groove 20 closest to the front end portion 17 will be the widest, while the width of the grooves behind will decrease, preferably gradually.

Preferably, all threads 19 in the threaded part 16 have crests with increasing width and preferably, all grooves 20 between the threads have a decreasing width.

Each thread 19 may have a front side which is inclined outwards/backwards relative to the front end portion 17 and the longitudinal axis L1 of the threaded part 16, and which defines or makes an angle of 70° ± 10% in relation thereto. Each thread 19 may also have a rear side which defines or makes an angle of 90° ± 5% in relation to the longitudinal axis L1. Hereby, the front sides 19a of the threads 19 become conical such that they facilitate screwing of the bone screw 6 into the bone material and their rear sides become transverse such that they can contribute to retain the bone screw 6 therein.

The width b1 of the narrowest crest 23 (on the thread 19 lying closest to the front end portion 17) can e.g. be 0,2 mm ± 10% and the width b7 of the widest crest 23 (on the thread 19 lying farthest away from the front end portion 17) can e.g. be 1,0 mm ± 10%.

The width B1 of the widest groove 20 (closest to the front end portion 17) can e.g. be 2,5 mm ± 10% and the width B6 of the narrowest groove 20 (lying farthest away from the front end portion 17) can e.g. be 2,0 mm ± 10%.

An example of a bone screw 6 with the above design or similar designs can have the following dimensions:
1) the length L of the threaded part 16 lies within the interval 21-25 mm and is preferably 23 mm ± 5%;
2) there are 6-9, preferably 8 complete threads 19 in the threaded part 16; and
3) the threads 19 have an outer diameter YD lying within the interval 6-13 mm ± 10%, preferably 8 mm ± 5%.

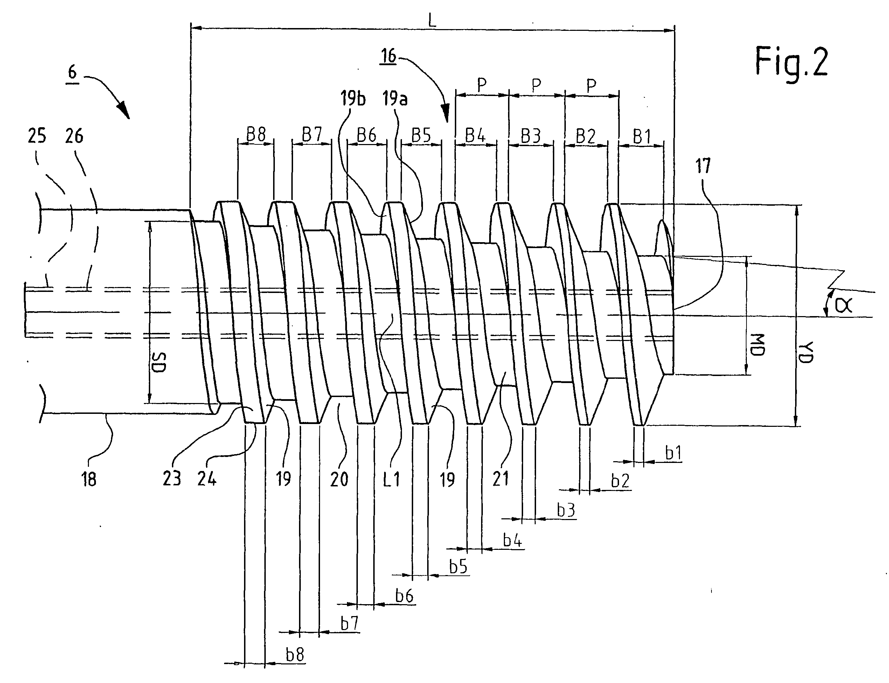

The bone screw 6 may either be solid, i.e. lack holes for a guide wire or have a longitudinal hole 25 for a guide wire 26. Said latter embodiment is indicated with broken lines in figure 2.

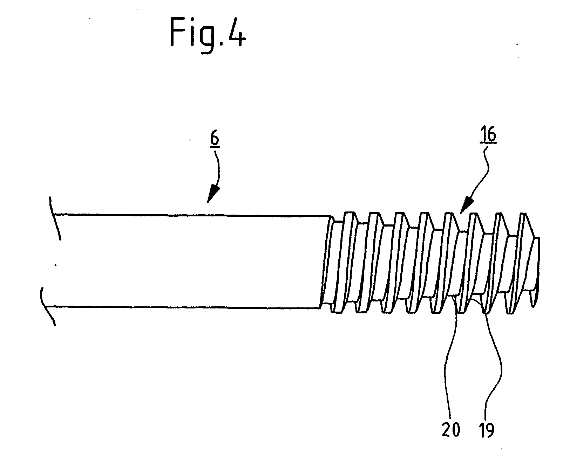

There may be different bone screws 6 with the above or similar construction for the right as well as the left leg. For the right leg, these bone screws may have right--hand threads (e.g. figure 2), while they for the left leg may have left-hand threads (figure 4).

Hereby, one can improve the ability of the bone screw 6 not to unscrew itself from the bone material because of the influence thereon by forces in different directions depending on whether they are provided in the right or in the left leg.

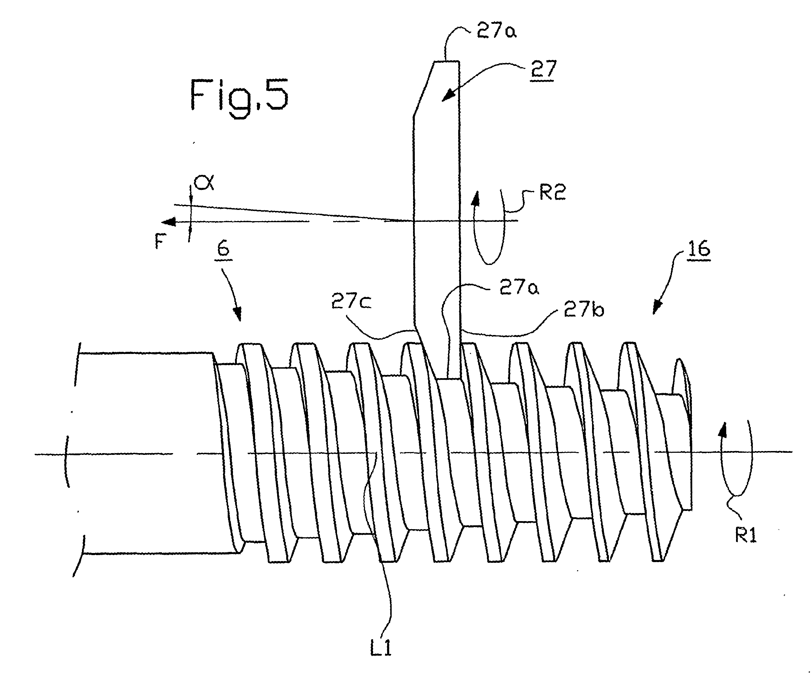

A way to produce the threaded part 16 or threaded parts on bone screws of another type is to mill the grooves 20 between the threads 19 by means of a milling tool 27, as is schematically illustrated in figure 5, said milling tool 27 lacking milling surfaces for milling the crests of the threads 19, i.e. a milling tool 27 by means of which it is possible to mill grooves 20 having different depths between said threads 19.

By means of the milling tool 27, grooves 20 are milled which are deepest at the front end portion 17 of the bone screw 6 and the depth of which decreases in backwards direction from said front end portion 17 to form the conical core 21 in the threaded part 16.

The milling tool 27 is brought to cut the threads 19 with a uniform, regular pitch. Also, the milling tool is brought to provide threads 19 having truncated crests 23, the width b1-b7 of which increases in backwards direction from the front end portion 17. Furthermore, the milling tool 27 is brought to provide grooves 20 between the threads 19, said grooves having a width B1-B6 which decreases in said backwards direction depending on the increase in width b1-b7 of the truncated crests 23.

The milling tool 27 has three milling surfaces, namely one milling surface 27a for milling the bottom of the grooves 20 between the threads 19, one milling surface 27b for the rear sides 19b of the threads 19 and one milling surface 27c for the front sides 19a of the threads 19.

During milling, the bone screw 6 rotates in the direction of rotation R1 and the milling tool 27 in the direction of rotation R2. The motion pattern in operation during milling may vary. Thus, the milling tool 27 can be moved substantially in an axial direction of movement F relative to the bone screw 6 or the bone screw 6 may eventually be moved in substantially the corresponding direction relative to the milling tool 27. For milling grooves 20 having different depths, the milling tool 27 can be moved in a sideways direction with an angle α relative to the bone screw 6 or the opposite, i.e. the angle α the longitudinal sides of the core 21 is making or defining with the longitudinal axis L1 of the threaded part 16.

In the embodiment shown, the threads of the bone screw 6 have a larger outer diameter YD than the untapped part 18 thereof. However, the outer diameter YD of the threads may be equal to the outer diameter of the untapped part, particularly if the bone screw shall be used in connection with intramedullary nails.

The front portion 15a of the drill 15 is preferably conical for drilling a conical hole 14 for the conical core 21 of the bone screw 6. The angle of inclination β between longitudinal lateral parts of the front portion 15a and the longitudinal axis L2 thereof corresponds with the angle of inclination α between the longitudinal lateral parts of the core 21 and the longitudinal axis L1 of the threaded part 16.

The invention is not limited to the embodiment of the bone screw described above and illustrated in the drawings, nor to the method for producing threads described above, but said embodiment and said method may vary within the scope of the subsequent claims.

It should also be mentioned that the bone screw 6 can be used at other fractures than trochanteric femoral fractures, such as humerus fractures, and it can be used at completely different bone implants than the one described and illustrated, e.g. at implants for external fixation.

## Claims

1. Bone screw for implants for fixation of bone fragments at fractures,
wherein the bone screw (6) comprises a threaded or tapped part (16) which is adapted to be screwed into a bone fragment (4) at the fracture (2),
wherein the threaded part (16) extends backwards from a front end portion (17) of the bone screw (6) to an untapped part (18) thereof,
wherein the threaded part (16) has threads with the same outer diameter (YD),
wherein the threads (19) in the threaded part (16) have the same pitch of thread (P), and
wherein the threads (19) in the threaded part (16) extend from a core (21),
**characterized in**
**that** at least front parts of the core (21) closest to the front end portion (17) of the bone screw (6) are conical and have their least diameter (MD) situated at said front end portion (17),
**that** at least some of the threads (19) have truncated crests (23),
**that** the width (b1-b7) of the crests (23) of at least some of the threads (19) increases in backwards direction from the front end portion (17), and
**that** the width (B1-B6) of grooves (20) between the threads (19) decreases in dependence of the increase in width (b1-b7) of the crests (23).

2. Bone screw according to claim 1, **characterized in**
**that** the width (b1-b7) of the crests (23) of all threads (19) or of at least the major part thereof increases gradually in backwards direction from the front end portion (17), and
**that** the width (B1-B6) of all grooves (20) between the threads (19) or of at least the major part thereof decreases gradually in backwards direction from said front end portion (17).

3. Bone screw according to any preceding claim, **characterized i n that** at least the major part of the number of threads (19), preferably all complete threads (19), have truncated crests (23).

4. Bone screw according to any preceding claim, **characterized in**
**that** each thread (19) has a front side (19a) which is inclined outwards/backwards relative to the longitudinal axis (L1) of the threaded part (16) and which defines or makes an angle of 70° ± 10% in relation thereto, and
**that** each thread (19) has a rear side (19b) which defines or makes an angle of 90° ± 5% relative to said longitudinal axis (L1).

5. Bone screw according to any preceding claim, **characterized in**
**that** the thread pitch (P) of the threads (19) lies within an interval of 3,2 at an outer diameter (YD) of the threads (19) of 12,7 mm and 2,6 at an outer diameter (YD) of 6,5 mm, and
**that** the thread pitch (P) preferably is 3,2 at an outer diameter (YD) of 12,7 mm ± 10%.

6. Bone screw according to any preceding claim, **characterized in**
**that** the width (b1) of the narrowest crest (23) is 0,2 mm ± 10% while the width (b7) of the widest crest (23) is 1,0 mm ± 10%, and
**that** the width (B1) of the widest groove (20) between the threads (19) is 2,5 mm ± 10% while the width (B6) of the narrowest groove (20) is 2,0 mm ± 10%.

7. Bone screw according to any preceding claim, **characterized in that** the core (21) is conical along the entire or at least substantial parts of the length (L) of the threaded part (16).

8. Bone screw according to claim 6, **characterized i n that** the diameter of the core (21) is increasing gradually in backwards direction from a least diameter (MD) at the front end portion (17) to a largest diameter (SD) at a transition (22) between the threaded or tapped part (16) and the untapped part (18) of the bone screw (6).

9. Bone screw according to any of claim 1 - 6, **characterized in that** the core (21) has a conical part closest to the front end portion (17), a cylindrical part behind said conical part and a conical part behind said cylindrical part.

10. Bone screw according to any preceding claim, **characterized in that** a longitudinal side of the core (21) or parts thereof forms an angle of inclination (α) of 2-4° ± 10%, preferably 3° ± 10%, with the longitudinal axis (L1) of the threaded part (16).

11. Bone screw according to any preceding claim, **characterized in**
**that** there are 6-9, preferably 8 complete threads (19) in the threaded part (16), and
**that** the threads (19) have an outer diameter of 6-13 mm ± 10%.

12. Bone screw according to any preceding claim, **characterized in that** it is solid in such a manner that it has no through hole for a guide wire in its longitudinal direction.

13. Bone screw according to any of claim 1 - 11, **characterized in that** it has a through hole (25) for a guide wire (26) in its longitudinal direction.

14. Bone screw according to any preceding claim, **chracterized in that** it is connectable to the bone implant (5) such that it after connection can be rotated in relation thereto.

15. Bone screw according to any preceding claim, **characterized in that** it is designed to fit into a sleeve (8) on the implant in the form of a bone implant, said sleeve (8) being adapted for insertion into a hole (11) provided in a bone fragment (4) on one side of the femoral fracture (2) and said sleeve (8) being connected to a plate (7) or similar with holes (9), preferably long holes, for screws (10) for securing the plate (7) to a bone fragment (3) on the other side of the femoral fracture (2).

16. Bone screw according to any preceding claim, **characterized in**
**that** a bone screw (6) for fixation of bone fragments (3, 4) at femoral fractures (2) on a right leg has right--hand threads, and
**that** a bone screw (6) for fixation of bone fragments (3, 4) at femoral fractures (2) on a left leg has left--hand threads.

17. Method for producing threads on a bone screw (6) according to any of claims 1 - 16,
**characterized by**
milling said grooves (20) between the threads (19) by means of a milling tool (27) which is designed for milling grooves (20) having different depths,
bringing said milling tool (27) to mill grooves (20) having the largest depth closest to a front end portion (17) of the bone screw and the depth of which decreases in backwards direction from said front end portion (17) for obtaining a conical core (21) from which the threads (19) extend,
bringing the milling tool (27) to cut threads (19) with uniform thread pitch,
bringing the milling tool (27) to provide threads (19) with truncated crests (23), the width of (b1-b7) of which increases in backwards direction from the front end portion (17), and
bringing the milling tool (27) to provide grooves (20) between the threads (19), the width (B1-B6) of which decreases in backwards direction from the front end portion (17) depending on the increase in width (b1-b7) of the truncated crests (23).

## Patentansprüche

1. Knochenschraube für Implantate zum Befestigen von Knochenfragmenten an Knochenbrüchen,
wobei die Knochenschraube (6) einen gewindeten Teil (16) aufweist, der zum Einschrauben in ein Knochenfragment (4) an einem Knochenbruch (2) angepasst ist,
wobei sich der gewindete Teil (16) von einem vorderen Endabschnitt (17) der Knochenschraube (6) nach hinten bis zu einem gewindelosen Teil (18) der Schraube erstreckt,
wobei der gewindete Teil (16) Gewindegänge mit demselben Außendurchmesser (YD) hat,
wobei die Gewindegänge (19) im gewindeten Teil (16) dieselbe Steigung (P) haben, und
wobei sich die Gewindegänge (19) im gewindeten Teil (16) von einem Kern (21) aus erstrecken,
**dadurch gekennzeichnet,**
**dass** zumindest die dem vorderen Endabschnitt (17) der Knochenschraube (6) am nächsten gelegenen vorderen Teile des Kerns (21) konisch sind und sich ihr kleinster Durchmesser (MD) am vorderen Endabschnitt (17) befindet,
**dass** zumindest einige der Gewindegänge (19) kegelstumpfförmige Gewindescheitel (23) haben,
**dass** die Breite (b1 - b7) der Scheitel (23) von zumindest einigen der Gewindegänge (19) vom vorderen Endabschnitt (17) nach hinten zu zunimmt, und
**dass** die Breite (B1 - B6) der Rillen (20) zwischen den Gewindegängen (19) abhängig von der Zunahme der Breite (b1 - b7) der Scheitel (23) abnimmt.

2. Knochenschraube nach Anspruch 1, **dadurch gekennzeichnet,**
**dass** die Breite (b1 - b7) der Scheitel (23) aller Gewindegänge (19) oder zumindest des größten Teil derselben vom vorderen Endabschnitt (17) nach hinten zu allmählich zunimmt, und
**dass** die Breite (B1 - B6) aller Rillen (20) zwischen den Gewindegängen (19) oder zumindest des größten Teil derselben vom vorderen Endabschnitt (17) nach hinten zu allmählich abnimmt.

3. Knochenschraube nach einem der vorangehenden Ansprüche, **dadurch gekennzeichnet, dass** zumindest der größte Teil der Gewindegänge (19), vorzugsweise alle vollständigen Gewindegänge (19), kegelstumpfförmige Scheitel (23) haben.

4. Knochenschraube nach einem der vorangehenden Ansprüche, **dadurch gekennzeichnet,**
**dass** jeder Gewindegang (19) eine Vorderseite (19a) hat, die relativ zur Längsachse (L1) des gewindeten Teils (16) nach außen/ hinten geneigt ist und mit ihr einen Winkel von 70° ± 10% einschließt oder bildet, und
**dass** jeder Gewindegang (19) eine Rückseite (19b) hat, die mit dieser Längsachse (L1) einen Winkel von 90° ± 5% einschließt oder bildet.

5. Knochenschraube nach einem der vorangehenden Ansprüche, **dadurch gekennzeichnet,**
**dass** die Steigung (P) der Gewindegänge (19) bei einem Außendurchmesser (YD) der Gewindegänge (19) von 12,7 mm innerhalb eines Intervalls von 3,2 und bei einem Außendurchmesser (YD) von 6,5 mm innerhalb von 2,6 liegt, und
**dass** die Steigung (P) bei einem Außendurchmesser (YD) von 12,7 mm ± 10% vorzugsweise 3,2 beträgt.

6. Knochenschraube nach einem der vorangehenden Ansprüche, **dadurch gekennzeichnet,**
**dass** die Breite (b1) des schmalsten Scheitels (23) 0,2 mm ± 10% beträgt, während die Breite (b7) des breitesten Scheitels (23) 1,0 mm ± 10% beträgt, und
**dass** die Breite (B1) der breitesten Rille (20) zwischen den Gewindegängen (19) 2,5 mm ± 10% beträgt, während die Breite (B6) der schmalsten Rille (20) 2,0 mm ± 10% beträgt.

7. Knochenschraube nach einem der vorangehenden Ansprüche, **dadurch gekennzeichnet, dass** der Kern (21) über seine gesamte Länge (L) des gewindeten Teils (16) oder zumindest wesentliche Teile derselben konisch ist.

8. Knochenschraube nach Anspruch 6, **dadurch gekennzeichnet, dass** der Durchmesser des Kerns (21) von einem kleinsten Durchmesser (MD) am vorderen Endabschnitt (17) bis zu einem größten Durchmesser (SD) am Übergang (22) zwischen dem gewindeten Teil (16) und dem gewindelosen Teil (18) der Knochenschraube (6) allmählich nach hinten zu zunimmt.

9. Knochenschraube nach einem der Ansprüche 1 - 6, **dadurch gekennzeichnet, dass** der Kern (21) an der dem vorderen Endabschnitt am nächsten gelegenen Stelle einen konischen Teil, hinter diesem konischen Teil einen zylindrischen Teil und hinter diesem zylindrischen Teil einen konischen Teil hat.

10. Knochenschraube nach einem der vorangehenden Ansprüche, **dadurch gekennzeichnet, dass** eine Längsseite des Kerns (21) oder Teile davon mit der Längsachse (L1) des gewindeten Teils (16) einen Neigungswinkel (α) von 2° - 4° ± 10%, vorzugsweise 3° ± 10%, bildet.

11. Knochenschraube nach einem der vorangehenden Ansprüche, **dadurch gekennzeichnet,**
**dass** der gewindete Teil (16) 6 - 9, vorzugsweise 8 vollständige Gewindegänge (19) aufweist, und
**dass** die Gewindegänge einen Außendurchmesser von 6 - 13 mm ± 10% haben.

12. Knochenschraube nach einem der vorangehenden Ansprüche, **dadurch gekennzeichnet, dass** sie massiv ist, in der Weise, dass sie in ihrer Längsrichtung kein Durchgangsloch für einen Führungsdraht hat.

13. Knochenschraube nach einem der Ansprüche 1 - 11, **dadurch gekennzeichnet, dass** sie in ihrer Längsrichtung ein Durchgangsloch (25) für einen Führungsdraht (26) hat.

14. Knochenschraube nach einem der vorangehenden Ansprüche, **dadurch gekennzeichnet, dass** sie so mit dem Knochenimplantat (5) verbunden werden kann, dass sie nach der Verbindung relativ zu diesem rotiert werden kann.

15. Knochenschraube nach einem der vorangehenden Ansprüche, **dadurch gekennzeichnet, dass** sie so konstruiert ist, dass sie in eine Muffe (8) auf dem Implantat in Form eines Knochenimplantats passt, wobei diese Muffe (8) zum Einführen in ein im Knochenfragment (4) auf einer Seite des Oberschenkelbruchs (2) vorgesehenes Loch (11) angepasst ist und die Muffe (8) verbunden ist mit einer Platte (7) oder Ähnlichem mit Löchern (9), vorzugsweise langen Löcher, für Schrauben (10) zum Befestigen der Platte (7) an einem Knochenfragment (3) auf der anderen Seite des Oberschenkelbruchs.

16. Knochenschraube nach einem der vorangehenden Ansprüche, **dadurch gekennzeichnet,**
**dass** eine Knochenschraube (6) zum Befestigen von Knochenfragmenten (3, 4) an Oberschenkelbrüchen (2) an einem rechten Bein ein Rechtsgewinde hat, und
**dass** eine Knochenschraube (6) zum Befestigen von Knochenfragmenten (3, 4) an Oberschenkelbrüchen (2) an einem linken Bein ein Linksgewinde hat.

17. Verfahren zum Herstellen von Gewindegängen an einer Knochenschraube (6) nach einem der Ansprüche 1-16,
**dadurch gekennzeichnet,**
**dass** die Rillen (20) zwischen den Gewindegängen (19) mittels eines Fräswerkzeugs (27), welches zum Fräsen von Rillen (20) mit unterschiedlichen Tiefen ausgelegt ist, gefräst werden,
**dass** das Fräswerkzeug (27) dazu gebracht wird, Rillen (20) zu fräsen, welche die größte Tiefe an der einem vorderen Endabschnitt (17) der Knochenschraube am nächsten gelegenen Stelle hat und deren Tiefe von diesem vorderen Endabschnitt (17) nach hinten zu abnimmt, um einen konischen Kern (21) zu erhalten, von dem aus sich die Gewindegänge (19) erstrecken,
**dass** das Fräswerkzeug (27) dazu gebracht wird, Gewindegänge (19) mit einheitlicher Gewindesteigung zu schneiden,
**dass** das Fräswerkzeug (27) dazu gebracht wird, Gewindegänge (19) mit kegelstumpfförmigen Scheiteln (23) zu erzeugen, deren Breite (b1 - b7) vom vorderen Endabschnitt (17) nach hinten zu zunimmt, und
**dass** das Fräswerkzeug (27) dazu gebracht wird, Rillen (20) zwischen den Gewindegängen (19) zu erzeugen, deren Breite (B1 - B6) abhängig von der Zunahme der Breite (b1 - b7) der kegelstumpfförmigen Scheitel vom vorderen Endabschnitt (17) nach hinten zu abnimmt.

## Revendications

1. Vis à os destinée à des implants à des fins de fixation de fragments d'os à des fractures,
dans laquelle la vis à os (6) comprend une partie filetée ou une partie taraudée (16) qui est conçue pour venir s'insérer par vissage dans un fragment d'os (4) à la fracture (2) ;
dans laquelle la partie filetée (16) s'étend vers l'arrière à partir d'une portion terminale frontale (17) de la vis à os (6) jusqu'à une partie (18) non taraudée de ladite vis ;
dans laquelle la partie filetée (16) possède des filets de vis possédant le même diamètre externe (YD) ;
dans laquelle les filets de vis (19) dans la partie filetée (16) possèdent le même pas de vis (P) ; et
dans laquelle les filets de vis (19) dans la partie filetée (16) s'étendent à partir d'une partie centrale (21) ;
**caractérisée**
**en ce que** au moins les parties frontales de la partie centrale (21) les plus proches de la portion terminale frontale (17) de la vis à os (6) sont coniques, leur plus petit diamètre (MD) étant situé à ladite portion terminale frontale (17) ;
**en ce que** au moins un certain nombre des filets de vis (19) possèdent des crêtes tronquées (23) ;
**en ce que** la largeur (b1-b7) des crêtes (23) d'au moins un certain nombre des filets de vis (19) augmente vers l'arrière à partir de la portion terminale frontale (17) ; et
**en ce que** la largeur (B1-B6) des rainures (20) entre les filets de vis (19) diminue de manière proportionnelle à l'augmentation de la largeur (b1-b7) des crêtes (23).

2. vis à os selon la revendication 1, **caractérisée**
**en ce que** la largeur (b1-b7) des crêtes (23) de tous les filets de vis (19) ou d'au moins la majeure partie de ces derniers augmente progressivement vers l'arrière à partir de la portion terminale frontale (17) ; et
**en ce que** la largeur (B1-B6) de toutes les rainures (20) entre les filets de vis (19) ou d'au moins la majeure partie desdites rainures diminue progressivement vers l'arrière à partir de ladite portion terminale frontale (17).

3. vis à os selon l'une quelconque des revendications précédentes, **caractérisée en ce que** au moins la majeure partie de l'ensemble des filets de vis (19), de préférence la totalité des filets de vis complets (19) possèdent des crêtes tronquées (23).

4. Vis à os selon l'une quelconque des revendications précédentes, **caractérisée**
**en ce que** chaque filet de vis (19) possède un côté avant (19a) qui est incliné vers l'extérieur/vers l'arrière par rapport à l'axe longitudinal (L1) de la partie filetée (16) et qui définit ou qui forme un angle de 70° ± 10 % par rapport audit axe ; et
**en ce que** chaque filet de vis (19) possède un côté arrière (19a) qui définit ou qui forme un angle de 90° ± 5 % par rapport audit axe longitudinal (L1).

5. Vis à os selon l'une quelconque des revendications précédentes, **caractérisée**
**en ce que** le pas de vis (P) des filets de vis (19) se situe dans un intervalle de 3,2 au diamètre externe (YD) des filets de vis (19) de 12,7 mm et de 2,6 au diamètre externe (YD) de 6,5 mm ; et
**en ce que** le pas de vis (P) s'élève de préférence à 3,2 au diamètre externe (YD) de 12,7 mm ± 10 %.

6. Vis à os selon l'une quelconque des revendications précédentes, **caractérisée**
**en ce que** la largeur (b1) de la crête la plus étroite (23) s'élève à 0,2 mm ± 10 %, tandis que la largeur (b7) de la crête la plus large (23) s'élève à 1,0 mm ± 10 % ; et
**en ce que** la largeur (B1) de la rainure la plus large (20) s'élève à 2,5 mm ± 10 %, tandis que la largeur (B6) de la rainure la plus étroite (23) s'élève à 2,0 mm ± 10 %.

7. Vis à os selon l'une quelconque des revendications précédentes, **caractérisée en ce que** la partie centrale (21) est conique sur la totalité ou au moins sur des parties importantes de la longueur (L) de la partie filetée (16).

8. vis à os selon l'une quelconque des revendications précédentes, **caractérisée en ce que** le diamètre de la partie centrale (21) augmente de manière progressive vers l'arrière à partir du plus petit diamètre (MD) à la portion terminale frontale (17) jusqu'au plus grand diamètre (SD) à la transition (22) entre la partie filetée ou la partie taraudée (16) et la partie non taraudée (18) de la vis à os (6).

9. vis à os selon l'une quelconque des revendications 1 à 6, **caractérisée en ce que** la partie centrale (21) possède une partie conique la plus proche de la portion terminale frontale (17), une partie cylindrique derrière ladite partie conique et une partie conique derrière ladite partie cylindrique.

10. vis à os selon l'une quelconque des revendications précédentes, **caractérisée en ce que** le côté longitudinal de la partie centrale (21) ou des partie dudit côté forme(nt) un angle d'inclinaison (α) de 2 à 4° ± 10 %, de préférence de 3° ± 10 %, avec l'axe longitudinal (L1) de la partie filetée (16).

11. vis à os selon l'une quelconque des revendications précédentes, **caractérisée**
**en ce qu'**il y a de 6 à 9, de préférence 8 filets de vis complets (19) dans la partie filetée (16) ; et
**en ce que** les filets de vis (19) possèdent un diamètre externe de 6 à 13 mm ± 10 %.

12. Vis à os selon l'une quelconque des revendications précédentes, **caractérisée en ce qu'**elle est pleine dans le sens où elle est exempte de trous de passage pour un fil guide dans sa direction longitudinale.

13. Vis à os selon l'une quelconque des revendications 1 à 11, **caractérisée en ce qu'**elle possède un trou de passage (25) pour un fil guide (26) dans sa direction longitudinale.

14. Vis à os selon l'une quelconque des revendications précédentes, **caractérisée en ce qu'**elle peut être reliée à l'implant osseux (5) de telle manière que, après la liaison, elle peut tourner par rapport à ce dernier.

15. Vis à os selon l'une quelconque des revendications précédentes, **caractérisée en ce qu'**elle est conçue pour venir s'insérer dans un manchon (8) sur l'implant sous la forme d'un implant osseux, ledit manchon (8) étant conçu à des fins d'insertion dans un trou (11) pratiqué dans un fragment d'os (4) d'un côté de la fracture fémorale (2) et ledit manchon (8) est relié à une plaque (7) ou analogues comportant des trous (9), de préférence des trous allongés, pour des vis (10) à des fins de fixation de la plaque (7) à un fragment d'os (3) de l'autre côté de la fracture fémorale (2).

16. Vis à os selon l'une quelconque des revendications précédentes, **caractérisée**
**en ce qu'**une vis à os (6) pour la fixation de fragments d'os (3, 4) à des fractures fémorales (2) sur la jambe droite possède des pas de vis droits ; et
**en ce qu'**une vis à os (6) pour la fixation de fragments d'os (3, 4) à des fractures fémorales (2) sur la jambe gauche possède des pas de vis gauches.

17. Procédé de réalisation de filet de vis sur une vis à os (6) selon l'une quelconque des revendications 1 à 16,
**caractérisé par** le fait de
réaliser par fraisage lesdites rainures (20) entre les filets de vis (19) au moyen d'une fraise (27) qui est conçue pour le fraisage de rainures (20) possédant des profondeurs différentes ;
amener ladite fraise (27) à réaliser par fraisage des rainures (20) dont la profondeur maximale est la plus proche de la portion terminale frontale (17) de la vis à os et dont la profondeur diminue vers l'arrière à partir de ladite portion terminale frontale (17) pour obtenir une partie centrale conique (21) à partir de laquelle s'étendent les filets de vis (19) ;
amener la fraise (27) à découper des filets de vis (19) qui possèdent un pas de vis uniforme ;
amener la fraise (27) à procurer des filets de vis (19) comportant des crêtes tronquées (23) dont la largeur (b1-b7) augmente vers l'arrière à partir de la portion terminale frontale (17) ; et
amener la fraise (27) à procurer des rainures (20) entre les filets de vis (19) dont la largeur (B1-B6) diminue vers l'arrière à partir de la portion terminale frontale (17) de manière proportionnelle à l'augmentation de la largeur (b1-b7) des crêtes tronquées (23).
